# FASCICULE DE BREVET EUROPEEN

(11) **EP 2 585 576 B1**
(45) Date de publication et mention de la délivrance du brevet: **14.05.2014**
(21) Numéro de dépôt: 11721055.9
(22) Date de dépôt: 24.05.2011
(51) Int. Cl.: C12M 1/22, B01L 3/00

(54) **BOITE DE PETRI COMPORTANT DES MOYENS DE VERROUILLAGE EN VUE DE LA CONSTITUTION D'UNE PILE**
PETRISCHALE MIT BLOCKIERVORRICHTUNG ZUR BILDUNG EINES STAPELS
PETRI DISH COMPRISING LOCKING MEANS FOR THE PURPOSE OF FORMING A STACK

(30) Priorité: 22.06.2010 FR 1054959
(43) Date de publication de la demande: 01.05.2013
(73) Titulaire: AES Chemunex, 35270 Combourg (FR)
(72) Inventeur: HUET, Stéphane, F-35170 Bruz (FR); THEPAUT, Jérôme, F-35190 Quebriac (FR); GINCHELEAU, Christophe, F-35000 Rennes (FR); SIMON, Frédéric, F-35270 Combourg (FR); REVERDY, Frank, F-35270 Meillac (FR)
(74) Mandataire: Regimbeau
(86) Numéro de dépôt international: PCT/EP2011/058452
(87) Numéro de publication internationale: WO 2011/160911

(56) Documents cités:
- GB-A- 2 117 788
- JP-A- 2003 047 459
- JP-A- 2003 102 463
- US-A1- 2002 045 245
- US-A1- 2006 240 549

## Description

La présente invention est relative à une Boite de Petri pour la culture de micro-organismes, qui est constituée de deux éléments, à savoir un réceptacle et un couvercle complémentaire et qui ont tous les deux une forme de révolution.

De telles boîtes de Petri sont largement utilisées dans les laboratoires notamment et ces boîtes sont prévues pour être superposées de manière à constituer des piles.

Ceci permet notamment aux opérateurs de constituer des lots différents de boîtes de Petri, par exemple de manière à les trier selon la nature de la culture qui y est pratiquée.

Les documents JP-2003 047459, JP-2003 102463, US-2002/045245 et US-4 160 700 illustrent l'état de la technique en la matière.

Toutefois, si cette fonction d'empilement des boîtes est possible, il n'existe pas à ce jour de moyen qui permette de retenir les boîtes attachées l'une à l'autre.

Aussi, jusqu'ici, la manipulation de boîtes de culture nécessite l'utilisation de récipients de transport ou de systèmes externes de fixation des boîtes entre elles.

Ainsi, il est courant que les utilisateurs fassent usage d'un élastique ou d'un ruban adhésif pour solidariser les boîtes entre elles.

On comprend que l'utilisation de moyens externes implique la maîtrise de leur qualité sanitaire, et donc de leur stérilisation.

Dans certains cas, ces contraintes de manipulation et de transport des boîtes de Petri sont résolues par l'utilisation d'un outil de transport jetable supplémentaire, permettant ainsi le déplacement d'un lot de boîtes d'un emplacement à un autre, ce qui implique, dans tous les cas de figure, un surcoût.

La présente invention à pour but de résoudre ces difficultés.

A cet effet, elle propose une boîte de Petri, qui est constituée de deux éléments, à savoir un réceptacle et un couvercle complémentaire, qui ont tous les deux une forme de révolution et qui sont chacun délimités par une paroi de fond et au moins un muret périphérique, que lesdits réceptacle et couvercle portant des moyens de verrouillage complémentaires agencés de telle sorte que, lors de la constitution d'une pile formée de la superposition d'au moins deux boites, les moyens de verrouillage portés par un premier élément d'une première boite puissent coopérer avec les moyens de verrouillage du second élément de la deuxième boite, les rendant ainsi solidaires l'une de l'autre, l'un desdits éléments comportant un muret périphérique qui s'étend au-dessus et au-dessous de la paroi de fond, tandis que l'autre est dimensionné de telle sorte que, lors de la constitution de ladite pile, ledit élément d'une première boite soit partiellement engagé dans l'autre élément d'une deuxième boite, dans un logement délimité par la paroi de fond et une partie dudit muret, caractérisée par le fait que lesdits moyens de verrouillage complémentaires sont aptes à coopérer par mouvement relatif à rotation d'une boîte par rapport à l'autre, que lesdits moyens de verrouillage sont portés par les murets périphériques et qu'ils consistent en des saillies.

Ainsi, conformément à l'invention, les éléments qui constituent chacune des boîtes de Petri intègrent en leur sein des moyens de verrouillage à une autre boîte.

Il est donc possible, grâce à leur présence, de constituer des piles de boîtes dans lesquelles chaque boîte est rendue solidaire de sa ou ses voisines de manière à constituer un ensemble manipulable sans autre moyen de liaison externe.

Selon des caractéristiques avantageuses et non limitatives de cette boîte :
- lesdites saillies sont au nombre d'au moins deux, sont équidistantes angulairement et s'étendent sur une fraction angulaire desdits murets ;
- lesdites saillies ont la forme de portions de nervures hélicoïdales complémentaires ;
- la paroi de fond d'un desdits éléments est bombée vers l'extérieur et est déformable élastiquement de manière à lui faire occuper une position sensiblement plane.

D'autres caractéristiques et avantages de la présente invention apparaitront à la lecture de la description détaillée qui va suivre d'un mode de réalisation préférentiel.

Cette description sera faite en référence aux dessins annexés dans lesquels :
- la figure 1 est une vue très schématique, selon une coupe diamétrale, d'une structure de boîte de Petri conforme à l'invention ;
- la figure 2 est une vue de côté du couvercle d'une telle boîte de Petri, tandis que la figure 3 est une vue agrandie de la zone repérée par un cercle à la figure précédente ;
- la figure 4 est une vue de dessus du couvercle de la figure 4, tandis que la figure 5 est une vue agrandie de la zone repérée par un cercle à la figure précédente ;
- la figure 6 est une vue de dessous d'un réceptacle d'une boîte de Petri selon l'invention, tandis que la figure 7 est une vue agrandie de la zone repérée par un cercle à la figure précédente ;
- la figure 8 est une vue en perspective du réceptacle de la figure 6, tandis que la figure 9 est une vue agrandie de la zone repérée par un cercle à la figure précédente ;
- les figures 10, 12, 15 et 18 sont des vues en coupe transversale du réceptacle d'une boîte B₁ et du couvercle d'une boîte B₂, dans différentes positions qui conduisent, finalement, à leur solidarisation ;
- les figures 11, 13, 14, 16, 17 et 19 sont des vues de détail des zones repérées par un cercle aux figures situées immédiatement au dessus.

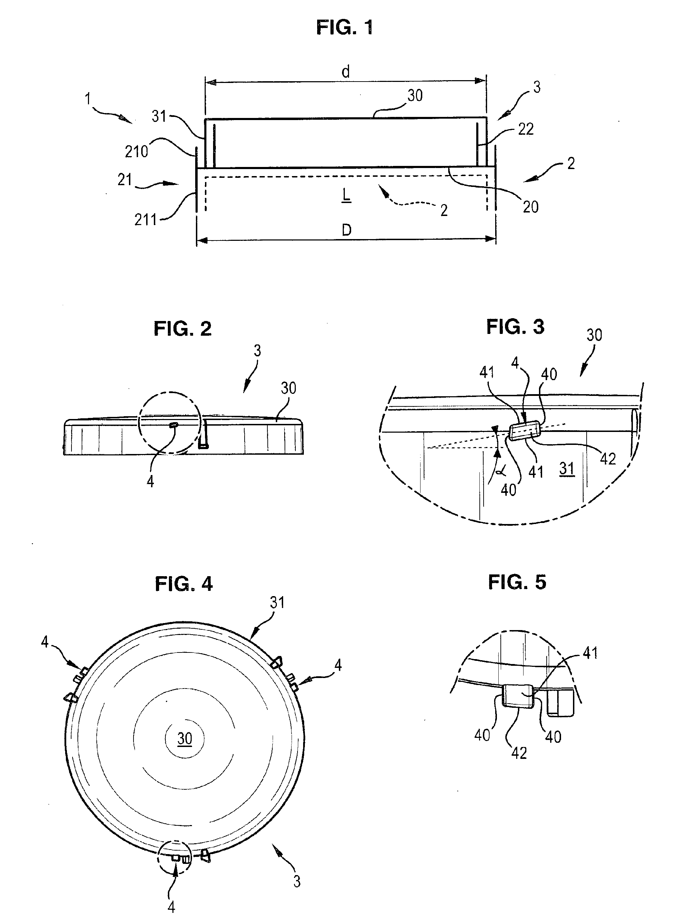

La présente invention s'applique tout particulièrement à une boîte de Petri telle que celle qui est représentée très schématiquement à la figure 1.

Comme toutes les boîtes de Petri, celle-ci est constituée de deux éléments, à savoir un réceptacle 2 et un couvercle complémentaire 3 et ont tous les deux une forme de révolution.

Ils sont par exemple réalisés en une matière plastique transparente telle que du polystyrène cristal.

Le réceptacle 2 comporte une paroi de fond 20 généralement plane qui est entourée par un muret périphérique 21.

Comme montré sur cette figure, ce muret s'étend à la fois au dessus et au dessous du plan dans lequel est contenu la paroi de fond 20, pour constituer une partie de muret supérieur 210 et une partie de muret inférieur 211.

Dans le mode de réalisation présenté, la paroi de fond 20 comporte également un autre muret 22, dit "muret intérieur", de sorte qu'il existe un espace annulaire entre les deux murets coaxiaux précités.

La paroi de fond 20 délimite avec le muret intérieur 22 l'espace dans lequel un milieu de culture est déposé.

Le couvercle 3 est constitué d'une paroi de fond 30 et d'un muret périphérique 31.

Ce dernier est dimensionné de manière à pouvoir être positionné contre la paroi de fond 20 du réceptacle 2, entre les murets 21 et 22.

Ces réceptacle 2 et couvercle 3 peuvent comporter des moyens non représentés qui permettent de les immobiliser l'un par rapport à l'autre.

Sous la lettre L est référencé, sur cette figure, un logement ouvert qui est délimité par la paroi de fond 20 du réceptacle et la partie inférieure 211 du muret 20.

Ce logement présente un diamètre **D**.

Le couvercle 3 est quant à lui dimensionné de manière à présenter un diamètre **d** tel que ce couvercle puisse être inséré dans l'espace annulaire situé entre les murets 21 et 22, comme déjà mentionné, mais de telle manière que le couvercle d'une autre boîte identique puisse être insérée dans le logement **L** précité.

C'est la situation présentée sur la figure 1 où le couvercle de cette seconde boîte est représenté sous la forme d'un profil en traits interrompus.

Cette configuration de boîte est une configuration traditionnelle.

Toutefois, dans certains modes de réalisation non représentés, la structure du réceptacle 2 et du couvercle 3 pourrait être inversée. Autrement dit, en basculant la figure 1 à 180°, le couvercle référencé 3 serait constitué par le réceptacle 2 et inversement.

Dans le mode de réalisation des figures 2 et suivantes, le couvercle 3 présente, sur son muret périphérique 31 des saillies ou tétons référencés 4.

On a affaire ici à un couvercle qui comporte trois saillies 4 identiques et équidistantes angulairement, ainsi que cela est bien visible à la figure 4.

Ces saillies s'étendent radialement vers l'extérieur en prenant naissance sur la face externe du muret 31.

Elles présentent deux faces supérieure et inférieure 41 parallèles et inclinées d'un angle aigu α par rapport à l'horizontale. Leurs deux faces latérales verticales sont référencées 40, et leur face d'extrémité 42.

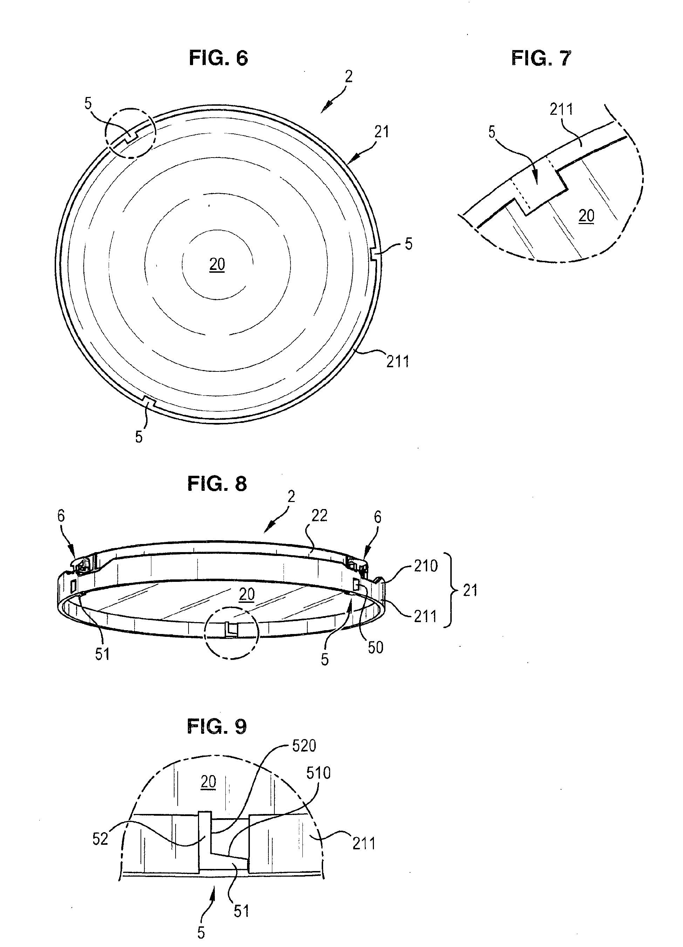

En se reportant maintenant aux figures 6 à 9, on constate que le réceptacle 2 comporte également le même nombre de saillies radiales complémentaires 5, qui sont portées par la partie inférieure 211 du muret 2 et tournées vers l'intérieur de la boîte.

Vues de face, ces saillies affectent une forme en L, avec une base 51 dont la face supérieure 510 est ascendante, et une branche verticale 52, dont la face 520 constitue une butée.

L'étendue transversale de ces saillies est prévue pour que, si l'on superpose un réceptacle 2 et un couvercle 3, les saillies complémentaires 4 et 5 interfèrent, quand on les rapproche.

En quelque sorte, les saillies 4 et 5 qui viennent d'être présentées ont la forme de portion de nervures hélicoïdales complémentaires.

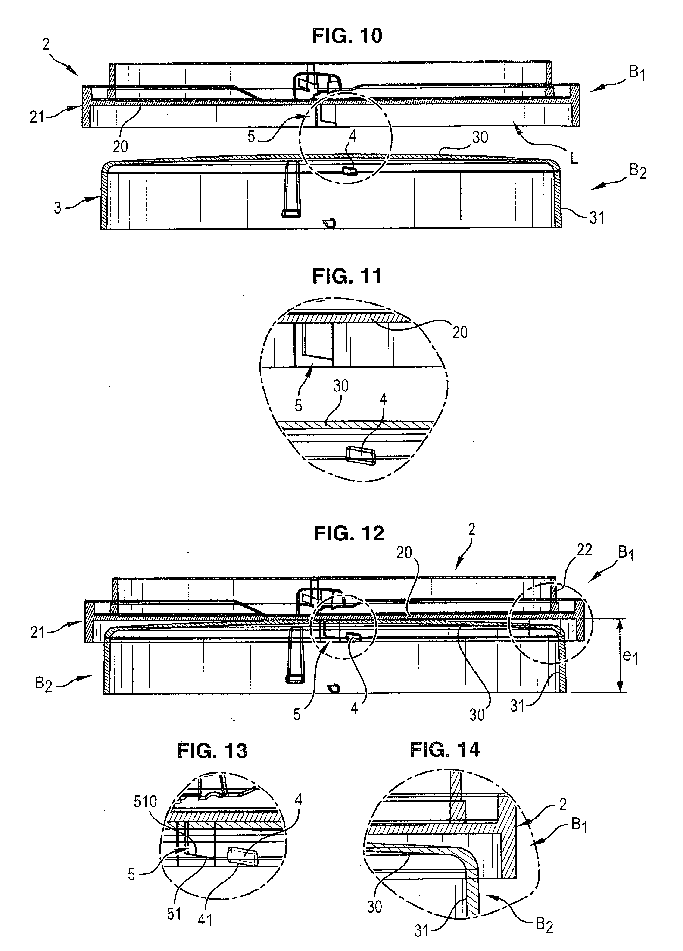

Aux figures 10 et 12 sont représentés le réceptacle 2 d'une première boîte de Petri B₁, ainsi que le couvercle 3 d'une seconde boîte de Petri B₂.

Ces figures montrent les positionnements relatifs de ces deux éléments, lorsqu'on souhaite les rendre solidaires l'un de l'autre.

Bien entendu, dans la pratique, le réceptacle 2 de la boîte B₁ est revêtu de son couvercle 3, tandis que le couvercle 3 de la boîte B₂ est assemblé à son réceptacle 2. Ces derniers n'ont toutefois pas été représentés sur les figures afin de ne pas les alourdir inutilement.

On notera, en référence à la figure 10, que la paroi de fond 30 du couvercle 3 est légèrement bombée, de convexité tournée vers l'extérieur dudit couvercle, cette paroi de fond présentant la capacité d'être déformable élastiquement, lorsqu'on exerce une pression sur cette dite paroi.

Autrement dit, soumise à une telle pression, elle présente la capacité, d'être ramenée dans une position où elle est sensiblement plane.

Il est à noter que sur les figures 10 et suivantes, les saillies 4 qui équipent le couvercle 3 sont vues par transparence, ce qui explique qu'elles présentent une orientation inversée par rapport à celle des figures 2 et 3, dans lesquelles elles sont vues depuis l'extérieur dudit couvercle.

En référence aux figures 10 et 11 et en vue de la constitution d'une pile de boîtes, deux boîtes B₁ et B₂ sont rapprochées l'une de l'autre de telle manière que le couvercle 3 de la boîte B₂ inférieure rentre partiellement dans le logement L du réceptacle 2 de la boîte B₁ supérieure, et que les saillies 4 et 5 ne coïncident pas verticalement (autrement dit ne se retrouvent pas à l'aplomb les unes des autres).

Ceci est représenté à la figure 10.

Ce mouvement de rapprochement est poursuivi jusqu'à ce que la paroi de fond 30 du couvercle 3 vienne en butée contre la paroi de fond 20 du réceptacle 2.

Dans cette position, le couvercle 2 présente une hauteur nominale e₁ et qui correspond à la hauteur cumulée du muret 31 et de la paroi de fond 30, du fait de sa forme légèrement courbe.

En se reportant aux figures 13 et 14, on constate alors que la face 510 de la saillie 5 se trouve à un niveau très légèrement supérieur à celui de la face inférieur 41 de la saillie 4, de sorte que même si on imprime à l'une des boîtes un mouvement de rotation l'une par rapport à l'autre, lesdites saillies ne peuvent pas coopérer mais, au contraire, viennent en butée l'une contre l'autre.

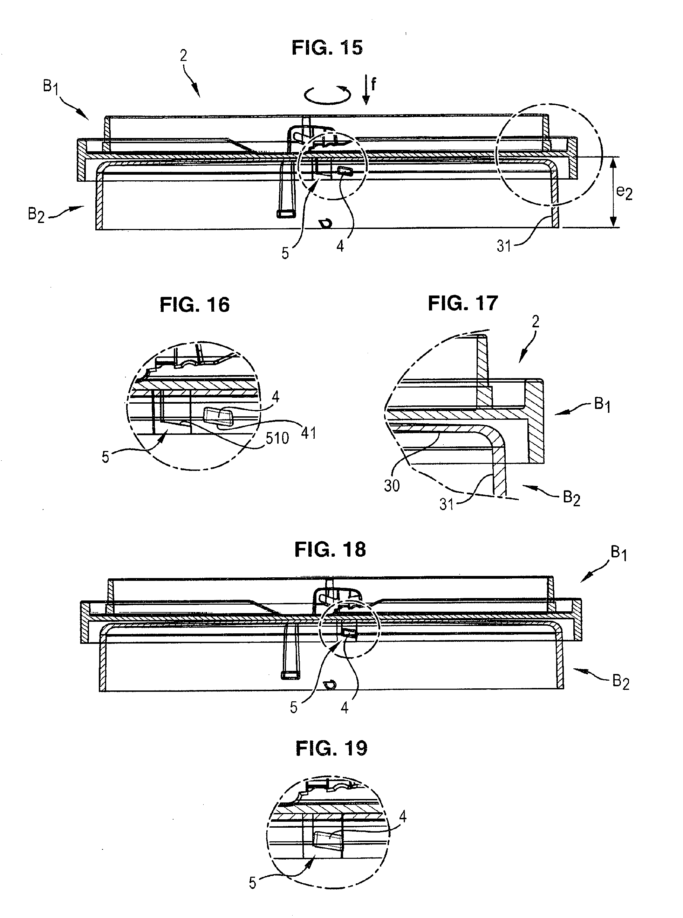

Par contre, et comme illustré à la figure 15 en opérant une pression sur la boîte supérieure B₁, on opère une déformation de la paroi de fond 30 du couvercle 3 de sorte que celui-ci occupe désormais une hauteur nominale e₂ inférieure à la hauteur e₁. Ce faisant, lesdites faces 510 et 41 se trouvent au même niveau, de sorte que par un mouvement de rotation d'une boîte par rapport à l'autre, on peut engager la saillie 4 dans la saillie 5.

L'orientation inclinée des faces 41 et 510, à la manière de portions de nervures hélicoïdales facilite cet engagement.

Bien entendu, l'homme du métier saura adapter les dimensions et le positionnement relatif des saillies 4 et 5 pour qu'elles coopèrent intimement.

On se trouve alors, au final, dans la situation de la figure 18 dans laquelle les deux saillies sont engagées l'une dans l'autre. On notera que la branche verticale de la saillie 5 constitue une butée qui empêche toute poursuite du mouvement de rotation d'une boîte par rapport à l'autre, de sorte que les saillies sont verrouillées dans cet état.

En opérant une traction sur une boîte par rapport à l'autre, dans une direction généralement perpendiculaire à leur paroi de fond, il est absolument impossible de les désolidariser.

Une telle désolidarisation ne peut être mise en oeuvre que volontairement, en opérant un mouvement rotatif dans le sens inverse de celui qui a été réalisé précédemment.

En faisant usage des moyens décrits ci-dessus, on peut donc constituer des piles de boîte de Petri qui sont rendues solidaires deux à deux, de sorte qu'il est envisageable de soulever une telle pile en saisissant simplement la boîte supérieure de l'empilage.

Bien entendu, la présente invention s'applique à toutes les structures de boîtes de Petri qui comportent des moyens de verrouillage complémentaires portés par les éléments constitués par le réceptacle et le couvercle.

Dans l'exemple décrit plus haut, la partie inférieure 211 du muret 21 du réceptacle 2 est prévue continue.

Toutefois, rien n'empêche qu'il présente certaines zones discontinuités notamment afin d'alléger au maximum ladite boîte de Petri.

## Revendications

1. Boite de Petri (1), qui est constituée de deux éléments, à savoir un réceptacle (2) et un couvercle complémentaire (3), qui ont tous les deux une forme de révolution et qui sont chacun délimités par une paroi de fond (20 ; 30) et au moins un muret périphérique (21 ; 31), que lesdits réceptacle (2) et couvercle (3) portant des moyens de verrouillage complémentaires (5 ; 4) agencés de telle sorte que, lors de la constitution d'une pile formée de la superposition d'au moins deux boites (B₁ ; B₂), les moyens (4 ; 5) de verrouillage portés par un premier élément (2 ; 3) d'une première boite (B₁) puissent coopérer avec les moyens de verrouillage (5 ; 4) du second élément (3 ; 2) de la deuxième boite (B₂), les rendant ainsi solidaires l'une de l'autre, l'un desdits éléments (2 ; 3) comportant un muret périphérique (21) qui s'étend au dessus et au-dessous de la paroi de fond (20), tandis que l'autre élément (3 ; 2) est dimensionné de telle sorte que, lors de la constitution de ladite pile, ledit élément (2 ; 3) d'une première boite (B₁) soit partiellement engagé dans l'autre élément (3 ; 2) d'une deuxième boite (B₂), dans un logement (L) délimité par la paroi de fond (20) et une partie (211) dudit muret (21), **caractérisé par le fait que** lesdits moyens de verrouillage complémentaires (5 ; 4) sont aptes à coopérer par mouvement relatif à rotation d'une boîte (B₁) par rapport à l'autre (B₂), que lesdits moyens de verrouillage sont portés par les murets périphériques (21 ; 31) et qu'ils consistent en des saillies (5 ; 4).

2. Boite selon la revendication 1, **caractérisée par le fait que** lesdites saillies (5 ; 4) sont au nombre d'au moins deux, sont équidistantes angulairement et s'étendent sur une fraction angulaire desdits murets (21 ; 31).

3. Boite selon l'une des revendications 1 ou 2, **caractérisée par le fait que** lesdites saillies (5 ; 4) ont la forme de portions de nervures hélicoïdales complémentaires.

4. Boite selon l'une des revendications précédentes, **caractérisée par le fait que** la paroi de fond (30) d'un (3) desdits éléments est bombée vers l'extérieur et est déformable élastiquement de manière à lui faire occuper une position sensiblement plane.

## Patentansprüche

1. Petrischale (1), die aus zwei Elementen besteht, nämlich einem Behälter (2) und einem dazu komplementären Deckel (3), die alle beide eine rotationssymmetrische Form haben und jeweils von einer Bodenwand (20;30) und mindestens einer Umfangswand (21;31) begrenzt sind, wobei der Behälter (2) und der Deckel (3) komplementäre Verriegelungsvorrichtungen (Blockiervorrichtungen) (5;4) aufweisen, die so konfiguriert sind, dass bei der Bildung eines Stapels, der aus mindestens zwei übereinander angeordneten Schalen (B₁;B₂) besteht, die Verriegelungsvorrichtungen (4;5), die von einem ersten Element (2;3) einer ersten Schale (B₁) getragen werden, mit den Verriegelungsvorrichtungen (5;4) des zweiten Elements (3;2) der zweiten Schale (B₂) zusammenwirken können und die Schalen dadurch fest miteinander verbunden werden, wobei eines der Elemente (2;3) eine Umfangswand (21) aufweist, die sich oberhalb und unterhalb der Bodenwand (20) erstreckt, während das andere Element (3;2) so bemessen ist, dass bei der Bildung des Stapels das Element (2;3) einer ersten Schale (B₁) teilweise in eine von der Bodenwand (20) und einem Teil (211) der Wand (21) begrenzte Aufnahme (L) des anderen Elements (3;2) einer zweiten Schale (B₂) eingesetzt wird, **dadurch gekennzeichnet, dass** die komplementären Verriegelungsvorrichtungen (5;4) durch eine Drehbewegung einer Schale (B₁) relativ zur anderen (B₂) zusammenwirken können, dass die Verriegelungsvorrichtungen von den Umfangswänden (21;31) getragen werden und dass sie aus Vorsprüngen (5;4) bestehen.

2. Petrischale nach Anspruch 1, **dadurch gekennzeichnet, dass** die Vorsprünge (5;4) mindestens zwei an der Zahl sind, äquidistant im Winkel angeordnet sind und sich über einen Winkelabschnitt der Wände (21;31) erstrecken.

3. Petrischale nach Anspruch 1 oder 2, **dadurch gekennzeichnet, dass** die Vorsprünge (5;4) die Form von Abschnitten komplementärer helikaler Rippen haben,

4. Petrischale nach einem der vorstehenden Ansprüche, **dadurch gekennzeichnet, dass** die Bodenwand (30) eines (3) der Elemente nach außen gewölbt ist und elastisch verformbar ist, um ihr zu ermöglichen, eine im Wesentlichen ebene Position einzunehmen.

## Claims

1. A Petri dish (1), which consists of two members, i.e. a receptacle (2) and an complementary lid (3), which both have a revolution shape and which are delimited by a bottom wall (20; 30) and at least one peripheral wall (21; 31), that said receptacle (2) and lid (3) bearing complementary locking means (5; 4) laid out so that, upon forming a stack formed with the superposition of at least two dishes (B₁; B₂), the locking means (4; 5) borne by a first member (2; 3) of a first dish (B₁) may cooperate with the locking means (5; 4) of the second member (3; 2) of the second dish (B₂), making them thus interdependent on each other, one of said members (2; 3) including a peripheral wall (21) which extends above and below the bottom wall (20) while the other member (3; 2) is dimensioned so that, upon forming said stack, said member (2; 3) of a first dish (B₁) is partly engaged into the other member (3; 2) of a second dish (B₂), in a housing (L) delimited by the bottom wall (20) and a portion (211) of said wall (21), **characterized by** the fact that said complementary locking means (5; 4) are able to cooperate by a relative rotary movement of one dish (B₁) relatively to the other one (B₂), that said locking means are borne by the peripheral walls (21; 31) and that they consist in protrusions (5; 4).

2. The dish according to claim 1, **characterized by** the fact that said protrusions (5; 4) are at least two in number, are angularly equidistant and extend over an angular fraction of said walls (21; 31).

3. The dish according to one of claims 1 or 2, **characterized by** the fact that said protrusions (5; 4) have the shape of portions of mating helicoidal ribs.

4. The dish according to one of the preceding claims, **characterized by** the fact that the bottom wall (30) of one (3) of said members bulges outwards and is elastically deformable so that it occupies a substantially planar position.
